# EUROPEAN PATENT SPECIFICATION

(11) **EP 3 890 671 B1**
(45) Date of publication and mention of the grant of the patent: **30.04.2025**
(21) Application number: 19828024.0
(22) Date of filing: 03.12.2019
(51) Int. Cl.: A61F 13/49, A61F 13/494, A61F 13/531

(54) **ABSORBENT ARTICLES HAVING AN ABSORBENT CORE CONFIGURED TO DEFINE A CONTAINMENT ZONE**
ABSORBIERENDER ARTIKEL MIT EINEM ABSORBIERENDEN KERN ZUM DEFINIEREN EINER SICHERHEITSZONE
ARTICLES ABSORBANTS COMPORTANT UN COEUR ABSORBANT CONFIGURÉ POUR DÉFINIR UNE ZONE DE CONFINEMENT

(30) Priority: 05.12.2018 US 201862775431 P
(43) Date of publication of application: 13.10.2021
(73) Proprietor: Attends Healthcare Products, Inc., Raleigh, NC 27617 (US)
(72) Inventor: CHMIELEWSKI, Harry, Raleigh, NC 27617 (US); SCHROER, Charles, F., Raleigh, NC 27617 (US)
(74) Representative: Lohr, Jöstingmeier & Partner
(86) International application number: PCT/US2019/064126
(87) International publication number: WO 2020/117731

(56) References cited:
- EP-A1- 1 447 065
- EP-A1- 1 457 185
- US-A1- 2006 241 557
- US-A1- 2006 264 861
- US-A1- 2007 197 987
- US-A1- 2015 245 958
- US-B1- 6 794 557

## Description

### CROSS-REFERENCE TO RELATED APPLICATIONS

This application claims priority to U.S. Provisional Application No. 62/775,431, filed December 5, 2018.

### FIELD OF INVENTION

The present invention relates generally to absorbent articles and, more particularly, to absorbent articles having an absorbent core configured to lift away from a chassis of the article to define a containment zone.

### BACKGROUND

Absorbent products can include, for example, baby diapers, training pants, and adult incontinence briefs and underwear, all of which may be made in disposable forms. "Disposable" refers to articles that are designed to be discarded after a limited use rather than being laundered or otherwise restored for reuse. Disposable absorbent products have met with widespread acceptance in the marketplace for a variety of applications, including infant and adult incontinence care, in view of the manner in which such products can provide effective and convenient liquid absorption and retention while maintaining the comfort of the wearer. Such disposable absorbent articles often include a topsheet that is configured to be closest to the wearer during use, a liquid-impermeable backsheet or outer cover, and an absorbent core between the topsheet and the backsheet. In some instances, such disposable absorbent articles also include an acquisition-distribution layer (ADL) disposed between the topsheet and the absorbent core. Elasticated standing leg cuffs and leg gathers are also often used in such articles to provide improved fit and reduced leakage around a wearer's legs, relative to articles without such cuffs or gathers.

EP 1 447 065 A1 discloses an absorbent sheet to be used, i.a., in a diaper and suggests to provide "a leakage prevention wall ... formed by an absorbent body" to avoid the costs for a leakage preventing elas-tic member (para. [0007]).

US 2015/245958 A1 relates to the field of absorbent laminates and multi-layer and suggests folded absorbent cores which define a longitudinally extending central channel to enhance liquid transport to lower layers of the folded core.

US 6,794,557 B1 as well suggests a disposable absorbent article with an absorbent core positioned on the upper surface of a chassis.

EP 1 457 185 A1 shows in FIG. 19 a folded structure having a top sheet 123, a back sheet 124 in between of a "*multilayer construction 120*" having a folded layer with blocks attached to it.

US 2006/241557 A1 suggests an absorbent articles with a first leakage preventing sheet on top of which is a non-folded absorbent core. A second leakage preventing sheet is located on the planar absorbent core.

U.S. Patent No. 4,670,011 discloses certain prior art examples of diapers, and U.S. Patents No. 6,976,978 and No. 4,940,464 disclose certain prior art examples of disposable incontinence garments or training pants.

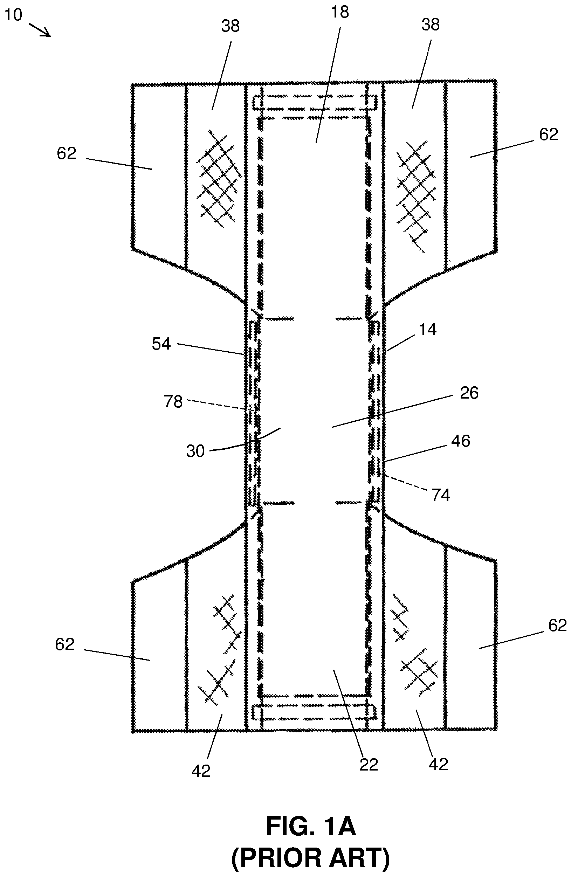

One example of such a disposable absorbent article is shown in FIGs. 1A-1B, which depict a lower plan view and a perspective view, respectively, of training pant 10. Training pant 10 includes a chassis 14 having a front waist portion 18, an opposing rear waist portion 22, and a crotch portion 26 extending longitudinally between front and rear waist portions 18, 22. Chassis 14 further includes a backsheet 30 defining an outer surface and configured to face away from a wearer during use of the diaper, and topsheet 34 defining an opposing body facing surface and configured to face a wearer during use of the diaper.

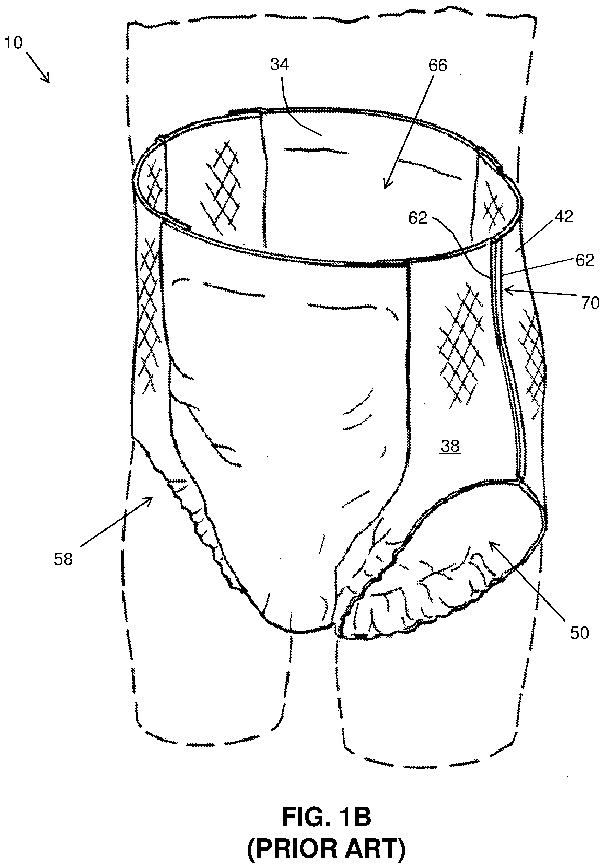

As shown in FIGS. 1A-1B, training pant 10 further includes a pair of front elastic side panels 38 and a pair of rear elastic side panels 42 configured to couple rear waist portion 22 to front waist portion 18 in a well-known configuration in which a left side 46 of the chassis defines a first leg opening 50 for a wearer's left leg, and in which a right side 54 of the chassis defines a second leg opening 58 for the wearer's right leg. In the depicted configuration, each of side panels 38, 42 includes a connection portion 62 configured to be coupled to a connection portion 62 of another of side panels 38, 42. Specifically, connection portion 62 of the left one of front side panels 38 is configure to be coupled to connection portion 62 of the left one of rear side panels 42, and connection portion 62 of the right one of front side panels 38 is configure to be coupled to connection portion 62 of the right one of rear side panels 42, such that the waist portions 18, 22 and side panels, 38, 42 cooperate to define a waist opening 66 as shown in FIG. 1B. Connection portions 62 of the respective side panels can be permanently coupled together to define a tear-able side seam 70, such as, for example, via adhesive, ultrasonic, or thermal bonds. Such tear-able side seams generally cannot be refastened, and thereby render an article unusable once opened. Alternatively, connection portions 62 of the respective side panels can be removably coupled to define a refastenable or adjustable side seam, such as, for example, via hook-and-loop fasteners. Hook and loop fasteners are mechanical fasteners that include hooks, such as in a hook fastener portion, that are configured to engage loops in a loop fastener portion or in fibers of a sheet of fabric; for example, a nonwoven or woven fabric with fibers that define open or loop-like regions into which the hooks can extend and engage. Examples of such hook and loop fasteners may be referred to as VELCRO.

As is known in the art, training pant 10 can include one or more elastic elements coupled to the chassis such that the one or more elastic elements resist expansion of a circumference of the first leg opening and resist expansion of a circumference of the second leg opening. For example, as shown in FIG. 1A, the depicted embodiment of chassis 14 includes a first elastic region 74 along right side 46, and a second elastic region 78 along left side 54. In some configurations, elastic regions 74, 78 can each be defined by one or more elastic strands, which may be referred to in the art as "leg elastics," coupled to the chassis, for example laminated between the topsheet or an additional leg cuff layer and the backsheet. In other configurations, elastic regions 74, 78 can each be defined by an elastic film coupled to the chassis, for example laminated between the topsheet and the backsheet. In configurations in which elastic regions 74, 78 are defined by elastic film, the regions can be defined by separate pieces of elastic film or by separate regions of a single piece of elastic film. As shown in FIG. 1A, elastic regions 74, 78 may be parallel to and/or extend along a majority of a length of each of sides 46 and 54, provided that the elastic regions are configured to provide a biasing force that resists expansion of the leg openings when the chassis is in its closed configuration and tends to contract the leg opening around a wearer's leg, as shown in FIG. 1B. Contraction of the leg opening to conform to the wearer's leg is desired for good containment of urine and feces in an absorbent product.

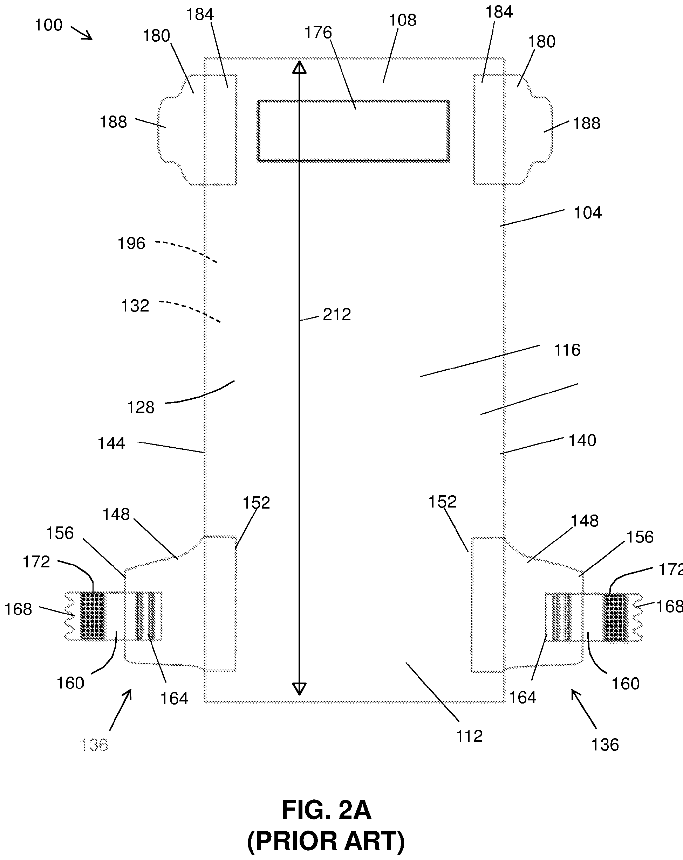

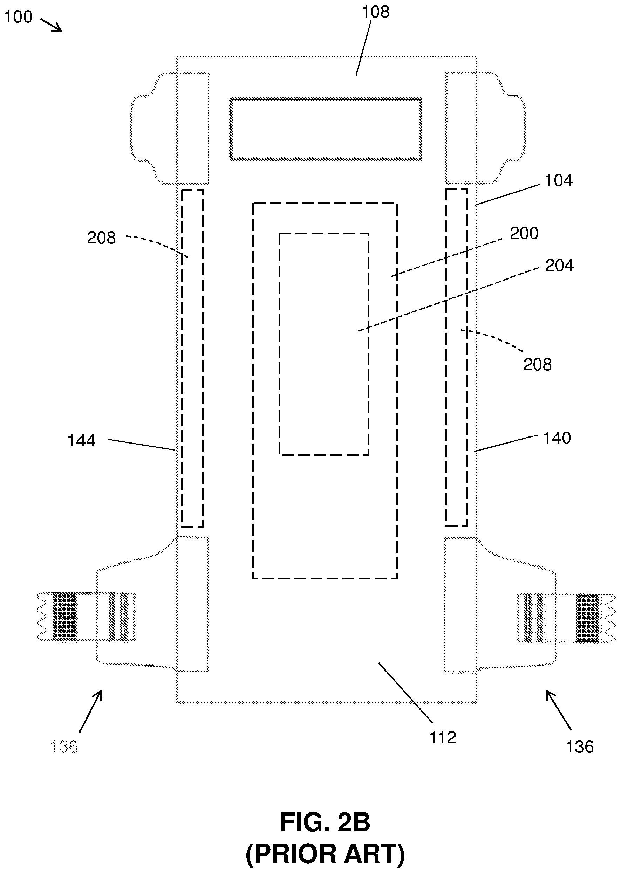

Another example of such a disposable absorbent article is shown in FIGs. 2A and 2B, which depict lower plan views of a baby diaper 100. Diaper 100 includes a chassis 104 having a front waist portion 108, an opposing rear waist portion 112, and a crotch portion 116 extending longitudinally between front and rear waist portions 108, 112. Chassis 104 further includes an outer surface 128 configured to face away from a wearer during use of the diaper, and an opposing body facing surface 132 configured to face a wearer during use of the diaper. In the view of FIG. 2A, a dashed leader extends from the body facing surface to reference numeral 132 because body facing surface 132 is opposite outer surface 128 and therefore not visible in the view of FIG. 2A.

As shown in FIG. 2A, diaper 100 further includes a pair of closure members 136 configured to couple rear waist portion 112 to front waist portion 108 in a well-known configuration in which a left side 140 of the chassis defines a first leg opening for a wearer's left leg, and in which a right side 144 of the chassis defines a second leg opening for the wearer's right leg, similar in some respects to what is shown in FIG. 1B for training pant 10. In the depicted configuration, the closure members include a pair of back ears or back ear panels 148 each having a first end 152 bonded to rear waist portion 112 of chassis 104, and a second end 156 shown extending away from rear waist portion 112. "Bonded" refers to the joining, adhering, connecting, attaching, or the like, of two elements via adhesive(s), ultrasonic bond(s), and/or thermal bond(s). Two elements will be considered to be bonded together when they are bonded directly to one another or indirectly to one another, such as when each is directly bonded to intermediate elements.

Each closure member 136 further includes a fastener tab 160 with a first end 164 bonded to back ear 148, a second end 168 shown extending laterally outward from back ear 148, and a fastener portion 172 coupled to the fastener tab. Back ears 148 are each formed of a stretchable elastic material, such as a nonwoven laminate, that permits adjustments in the width and tension of back ears 148 to vary the form and fit of diaper 100 when worn by a user.

Fastener tabs 160 are formed of an inelastic nonwoven material and carry fastener portions 172. Fastener portions 172 include strips of hook material configured to interact with a corresponding loop material in the well-known hook-and-loop fastener arrangement. Connection of closure members 136 to front waist portion 108 is facilitated by a landing zone 176 configured to be engaged by fastener portions 172. In this embodiment, landing zone 176 is defined by an anchoring member that includes a strip of loop material bonded to front waist portion 108 of chassis 104, for example, to the backsheet, and configured to be engaged by the hook material of fastener portions 172.

As shown in FIG. 2A, diaper 100 also includes a pair of front ears 180 extending from opposite sides 140, 144 of chassis 104 with each of front ears 180 each having a first end 184 bonded to front waist portion 108 of chassis 104, and a second end 188 shown extending away from a respective side of front waist portion 108. Front ears 180 are each formed of a relatively soft nonwoven material and are each configured to be overlapped by the corresponding fastener tab 160 and/or back ear 148 to prevent the edges of fastener tab 160 from pinching, rubbing, or otherwise irritating a user's skin in use when fastening portions 172 are engaged with landing zone 176 to couple rear waist portion 112 to front waist portion 108. In some embodiments, front ears 180 include loop fastener portions or a fabric that is configured to be engaged by hook fastener portions such that fastener portions 172a can engage front ears 180.

Outer surface 128 is defined by a liquid-impermeable backsheet or cover 192 that defines outer surface 128, and a liquid-permeable topsheet 196 that defines body facing surface 132 and is configured to be closest to the wearer during use. "Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. "Lamination" is the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are permanently assembled by heat, pressure, ultrasonic welding, or adhesives.

As shown in FIG. 2B, the depicted embodiment include an absorbent core 200 disposed between topsheet 196 and backsheet 192. An "absorbent core" is a structure typically disposed between a topsheet and backsheet of an absorbent article and containing materials like SAP and/or cellulosic fibers that are configured to absorb liquid in the absorbent article.

As shown in FIG. 2B, diaper 100 also includes an acquisition-distribution layer (ADL) 204 disposed between the topsheet and the absorbent core. "Layer" when used in the singular can be a single element or a plurality of elements. For example, a plurality of sheets may together define a single layer, such as, for example, a layer with a particular function to which the sheets of the layer contribute.

As is known in the art, diaper 100 can include one or more elastic elements coupled to the chassis such that the one or more elastic elements resist expansion of a circumference of the first leg opening and resist expansion of a circumference of the second leg opening. For example, as shown in FIG. 2B, the depicted configuration of chassis 104 includes a first elastic region 208 along first side 140, and a second elastic region 208 along second side 140. In some configurations, elastic regions 208 can each be defined by one or more elastic strands, which may be referred to in the art as "leg elastics," coupled to the chassis, for example laminated between the topsheet (or an additional leg cuff layer) and the backsheet. In other configurations, elastic regions 208 can each be defined by an elastic film coupled to the chassis, for example laminated between the topsheet (or an additional leg cuff layer) and the backsheet. In configurations in which elastic regions 208 are defined by elastic film, the regions can be defined by separate pieces of elastic film or by separate regions of a single piece of elastic film. As shown in FIG. 2B, elastic regions 208 may be parallel to and/or extend along a majority of a length of each of sides 140 and 144, provided that the elastic regions are configured to provide a biasing force that resists expansion of the leg openings when the chassis is in its closed configuration.

As shown in FIG. 2A, chassis 104 has an overall relaxed length 212.

Diaper 100 of FIGs. 2A and 2B is typically packaged and sold in a folded, and unfastened configuration in which chassis 104 is folded in half such that rear waist portion 108 overlaps front waist portion 104, but fastener portions 172 do not engage landing zone 176. While diaper 100 is described as a baby diaper, diaper 100 can also comprise an adult incontinence brief or training pant.

Conventional absorbent articles (e.g., training pant 10 and diaper 100) often fail to provide adequate containment of liquids and/or feces. Prior designs sometimes incorporate leg cuffs on either side of a flat absorbent core to mitigate leakage. Such leg cuffs may not have sufficient hydrostatic head to repel excess liquids and/or feces that are not yet absorbed by the flat absorbent core. Liquids and/or feces can also escape by breaking the seal between the leg cuff and a wearer's leg. As a result, liquids and/or feces may leak from the article and cause discomfort for a wearer. There accordingly is a need in the art for absorbent articles that have improved liquid and/or feces containment.

### SUMMARY

The present invention relates to an absorbent article defined by the subject-matter of claim 1.

The present absorbent articles address the need for improved liquid and/or feces containment by incorporating an absorbent core that can define a leakage-mitigating containment zone. The present cores can comprise a laminate longitudinally folded such that each of first and second longitudinally-extending edge regions of the core includes one or more folded laminate layers disposed on a lower laminate layer. At least a portion of the core in each of the edge regions is not bonded to a chassis and thus can lift away from the chassis to define the containment zone. The lifted portions provide an absorbent barrier that impedes the escape of liquid and/or feces, thereby eliminating the need for leg cuffs constructed from high hydrostatic head materials. Liquids and/or feces that escape the containment zone can be absorbed from underneath the lifted portions of the core to further mitigate leakage. Such lifting can be facilitated by cuffs that are bonded to at least one of the folded layers and configured to urge the free portions of the core away from the chassis. The cuffs can also at least partially unfold the laminate to increase the volume of the containment zone.

The articles comprise a chassis having opposing front and rear portions and a crotch portion extending longitudinally between the front a rear portions. In some articles, the chassis is configured to define a wearable configuration. In the articles, the crotch portion is configured to conform about at least one of a wearer's groin area, perineum, and rear when the chassis is in a wearable configuration. In some articles, a first end of the front portion is configured to be coupled to a first end of the rear portion and a second end of the front portion is configured to be coupled to a second send of the rear portion to define the wearable configuration. In such articles, the front and rear portions cooperate to encircle and define a waist opening, a left side of the chassis defines a first leg opening, and a right side of the chassis defines a second leg opening when the chassis is in the wearable configuration.

The articles have an absorbent core coupled to the chassis. The absorbent core extends longitudinally along the crotch portion and comprises a laminate. The laminate includes two or more substrate laminae and one or more absorbent laminae. Each of the absorbent lamina(e) comprises superabsorbent polymer (SAP). In some articles, the SAP of each of the absorbent lamina(e) has a basis weight between 60 and 75 grams per square meter (gsm) and, optionally, has a centrifuge retention capacity between 33 and 52 grams per gram (g/g). In some articles, the SAP of each of the absorbent lamina(e) comprises at least 90%, by weight, SAP. A first one of the absorbent lamina(e) is disposed between first and second ones of the substrate laminae. The first substrate lamina, in some embodiments, comprises tissue and, optionally, the second substrate lamina comprises a nonwoven. Some articles comprise three or more substrate laminae and two or more absorbent laminae, where a second one of the absorbent laminae is disposed between the second substrate lamina and a third one of the substrate laminae.

In the articles, the laminate is longitudinally folded such that the absorbent core includes a lower layer of the laminate and, within each of first and second longitudinally extending edge regions, one or more folded layers. The lower layer spans a lateral width of the absorbent core and each of the edge regions spans less than 50% , and optionally at least 20%, of the width of the absorbent core. In some articles, the one or more folded layers in each of the first and second edge regions comprise two or more folded layers. In some of such articles, for each of the first and second regions, a first one of the folded layers is bonded to a second one of the folded layers, optionally with an adhesive. In some articles, the laminate is folded such that, for each of the first and second edge regions, one of the folded layers extends inwardly from a fold of the laminate to an end of the laminate such that the end is disposed closer to the other of the edge regions than is the fold and, optionally, the folded layer is disposed below at least one other of the folded layers. In some articles, the laminate is folded such that, for the lower layer, the second substrate lamina is configured to be disposed closer to a wearer than is the first substrate lamina when the absorbent article is worn.

In the articles, a center region of the lower layer is bonded to the crotch portion of the chassis. The center region spans a lateral distance smaller than the width of the absorbent core such that at least a portion of the absorbent core in each of the edge regions is configured to lift away from the chassis when the chassis is in the wearable configuration. In some articles, a lowermost one of the folded layer(s) has and extends laterally between first and second portions. In some of such articles, the first portion is disposed closer to the other of the edge regions than is the second portion and, in some articles, the first portion is configured to be disposed on the lower layer when the chassis is in the wearable configuration.

Some articles have first and second longitudinally-extending cuffs. In some articles, each of the cuffs has a first portion coupled to the crotch portion. In some articles, each of the cuffs has a second portion bonded to at least one of the folded layer(s) of a respective one of the first and second edge regions. In some articles, for each of the cuffs, the second portion is configured to lift away from the chassis when the chassis is in the wearable configuration such that the cuff urges at least a portion of the absorbent core away from the chassis. In some articles, each of the cuffs is bonded to an uppermost one of the folded layer(s) of a respective one of the first and second end regions and, optionally, is not bonded to the chassis. In some of such articles, for each of the cuffs, the cuff is bonded to the uppermost one of the folded layer(s) such that a first side is disposed closer to the other of the edge regions than is a second side of the cuff. In some of such articles, for each of the cuffs, the second side of the cuff is configured to lift away from the chassis when the chassis is in the wearable configuration such that the second side is further from the chassis than is the first side and the cuff urges at least a portion of the absorbent core away from the chassis.

In some articles, the absorbent core has first and second ends and extends longitudinally between the first and second ends. Some of such articles have, for each of the first and second ends, an end cap that is coupled to the chassis and extends laterally across the end.

Some articles have a longitudinally-extending insert coupled to the laminate. In some of such articles, for each of the first and second edge regions, a portion of the insert is disposed above the lower layer and/or below at least one of the folded layer(s). In some articles, the insert comprises fluff and SAP and, additionally or alternatively, a through-air bonded polymer nonwoven.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified - and includes what is specified; e.g., substantially 90 degrees includes 90 degrees and substantially parallel includes parallel - as understood by a person of ordinary skill in the art. In any disclosed embodiment, the term "substantially" may be substituted with "within [a percentage] of" what is specified, where the percentage includes 0.1, 1, 5, and 10 percent.

The terms "comprise" and any form thereof such as "comprises" and "comprising," "have" and any form thereof such as "has" and "having," and "include" and any form thereof such as "includes" and "including" are open-ended linking verbs. As a result, an apparatus that "comprises," "has," or "includes" one or more elements possesses those one or more elements, but is not limited to possessing only those elements. Likewise, a method that "comprises," "has," or "includes" one or more steps possesses those one or more steps, but is not limited to possessing only those one or more steps.

Any embodiment of any of the apparatuses, systems, and methods can consist of or consist essentially of - rather than comprise/include/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

Further, a device or system that is configured in a certain way is configured in at least that way, but it can also be configured in other ways than those specifically described.

The feature or features of one embodiment may be applied to other embodiments, even though not described or illustrated, unless expressly prohibited by this disclosure or the nature of the embodiments.

Some details associated with the embodiments described above and others are described below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. Views in the figures are drawn to scale, unless otherwise noted, meaning the sizes of the depicted elements are accurate relative to each other for at least the embodiment in the view.
**FIG. 1A** is a bottom plan view of a prior art disposable absorbent article, specifically a training pant, in an open configuration.
**FIG. 1B** is a perspective view of the training pant of FIG. 1A in a closed configuration.
**FIG. 2A** is a bottom plan view of a prior art disposable absorbent article, specifically a baby diaper, in an open configuration.
**FIG. 2B** is a bottom plan view of the baby diaper of FIG. 2A, in an open configuration, showing certain internal components of the diaper.

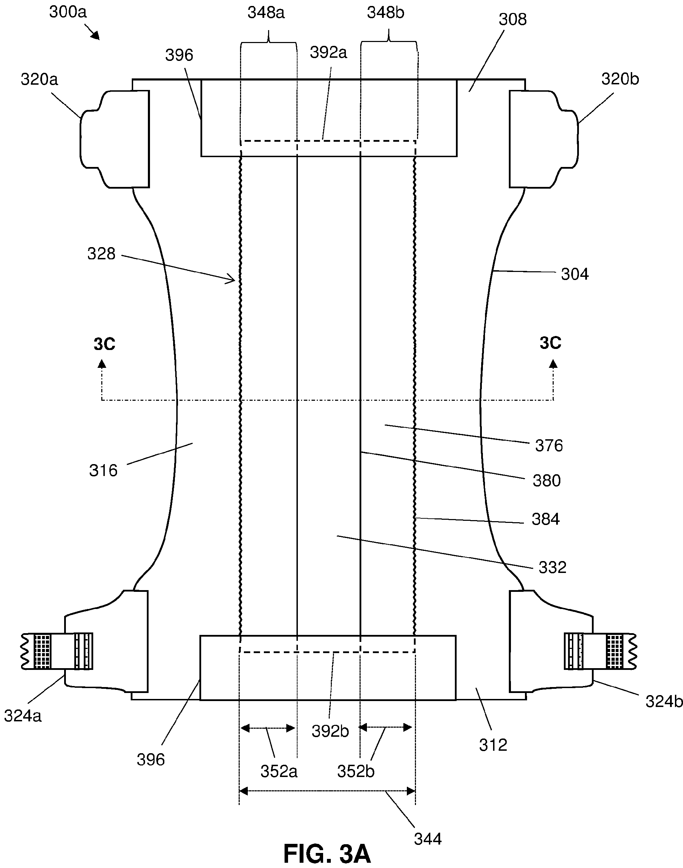

**FIG. 3A** is top plan view of a first embodiment of the present absorbent articles when its chassis is in an extended configuration. The article includes an absorbent core having, within each of first and second longitudinally-extending edge regions, one or more folded laminate layers disposed above a lower laminate layer and a cuff bonded to at least one of the folded layer(s). FIG. 3A is not necessarily to scale.

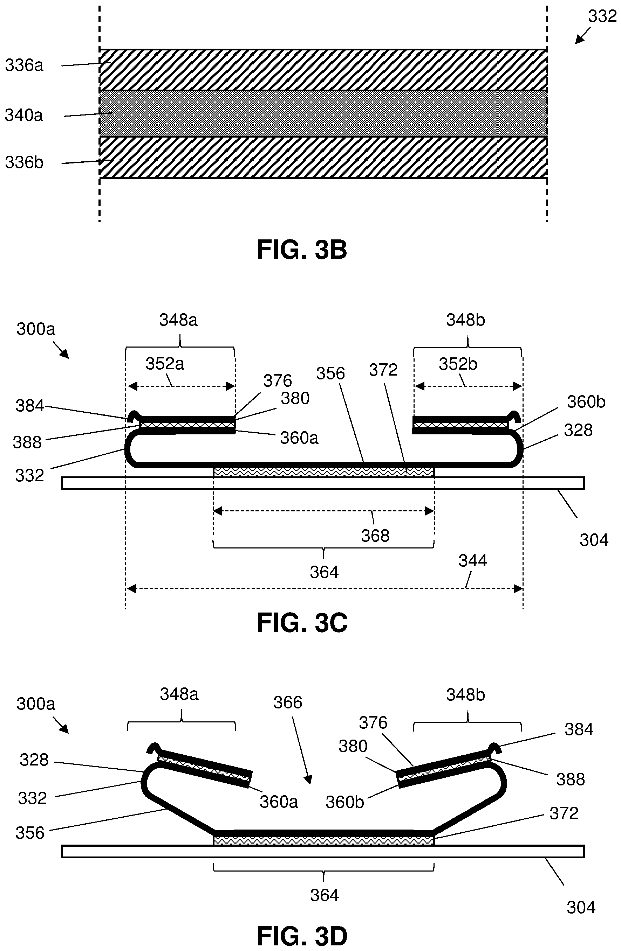

**FIG. 3B** is a schematic partial sectional view of a laminate suitable for use in the absorbent core of the article of FIG. 3A. FIG. 3B is not necessarily to scale.
**FIG. 3C** is a schematic sectional view of the article of FIG. 3A taken along line 3C-3C and illustrates the folded construction of the absorbent core when the chassis is in the extended configuration. FIG. 3C is not necessarily to scale.
**FIG. 3D** is a schematic sectional view of the article of FIG. 3A when its chassis is in a wearable configuration and illustrates the manner in which cuffs and free portions of the core lift away from the chassis to define a containment zone. FIG. 3D is not necessarily to scale.

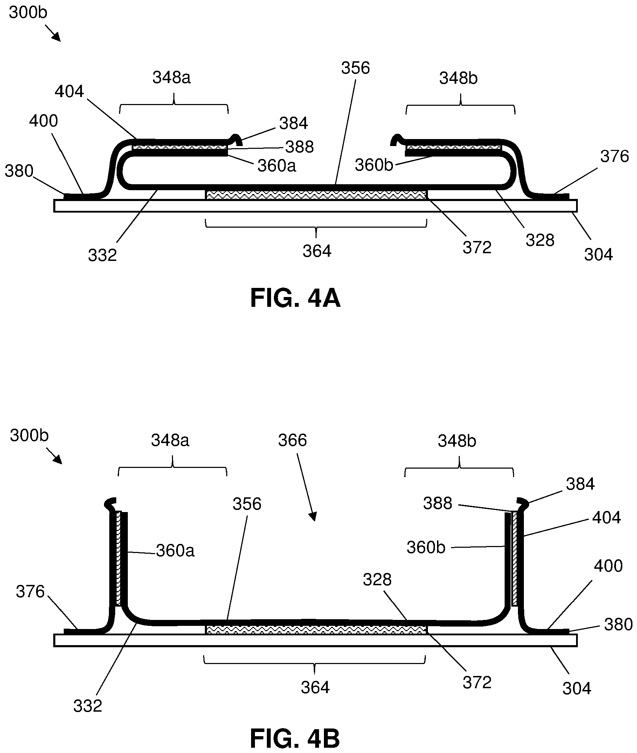

**FIG. 4A** is a schematic sectional view of a second embodiment of the present absorbent articles when its chassis is in an extended configuration. The second embodiment is substantially similar to the article of FIG. 3A, except that each of the cuffs is bonded both to at least one of the folded layers and to the chassis. FIG. 4A is not necessarily to scale.
**FIG. 4B** is a schematic sectional view of the article of FIG. 4A when its chassis is in a wearable configuration and illustrates the manner in which the cuffs and free portions of the core lift away from the chassis to define a containment zone. FIG. 4B is not necessarily to scale.

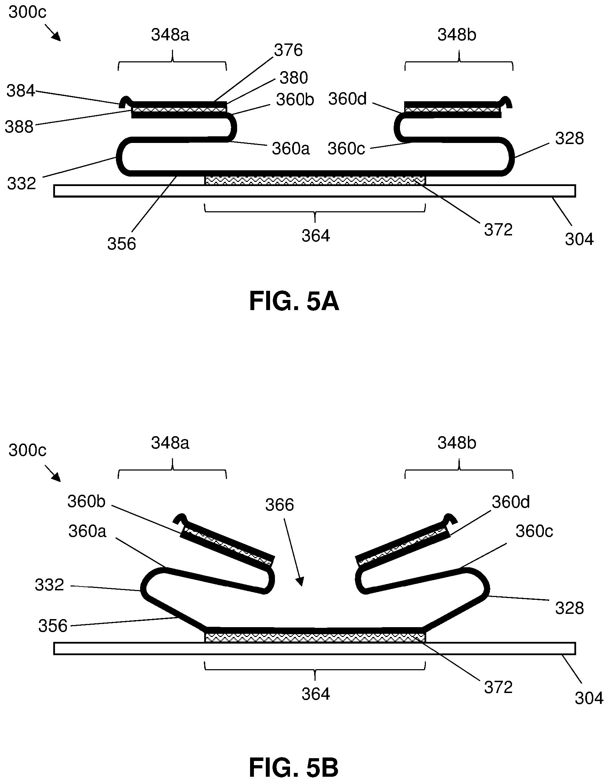

**FIG. 5A** is a schematic sectional view of a third embodiment of the present absorbent articles when its chassis is in an extended configuration. The third embodiment is substantially similar to the article of FIG. 3A, except that each of the edge regions has multiple folded layers. FIG. 5A is not necessarily to scale.
**FIG. 5B** is a schematic sectional view of the article of FIG. 5A when its chassis is in a wearable configuration and illustrates the manner in which the cuffs and free portions of the core lift away from the chassis to define a containment zone. FIG. 5B is not necessarily to scale.

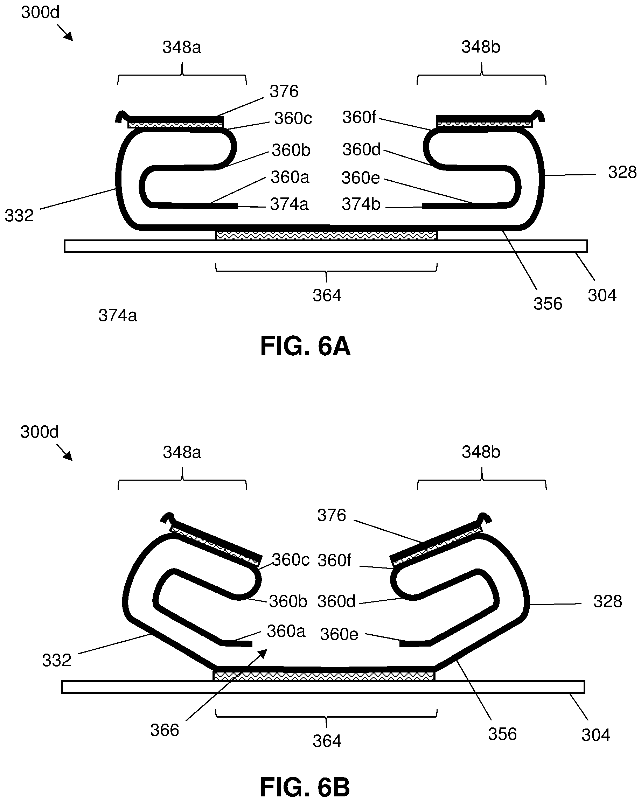

**FIG. 6A** is a schematic sectional view of a fourth embodiment of the present absorbent articles when its chassis is in an extended configuration. The fourth embodiment is substantially similar to the article of FIG. 3A, except that the laminate is folded such that, for each of the edge regions, there are multiple folded layers and at least one of the folded layers is configured to create a longitudinal dam. FIG. 6A is not necessarily to scale.
**FIG. 6B** is a schematic sectional view of the article of FIG. 6A when its chassis is in a wearable configuration and illustrates the manner in which, for each of the edge regions, at least one of the folded layers creates a longitudinal dam when the cuffs and free portions of the core lift away from the chassis. FIG. 6B is not necessarily to scale.

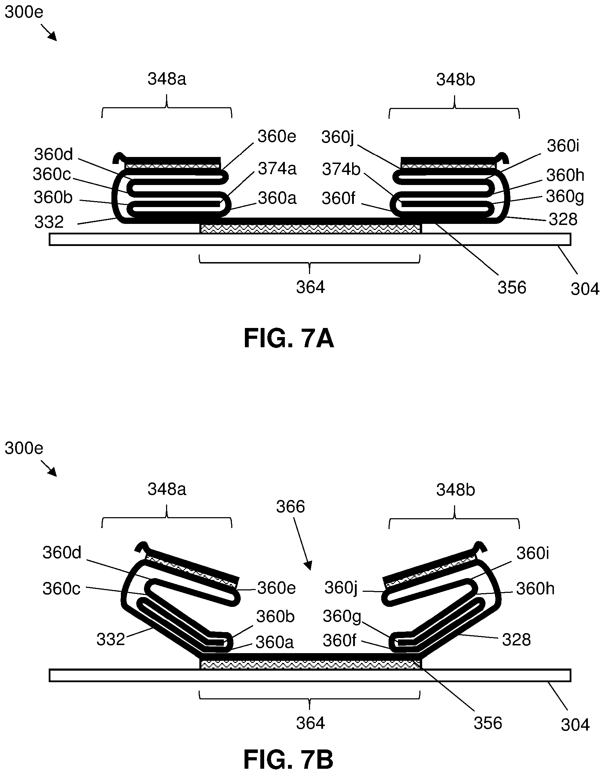

**FIG. 7A** is a schematic sectional view of a fifth embodiment of the present absorbent articles when its chassis is in an extended configuration. The fifth embodiment is substantially similar to the article of FIG. 3A, except that the laminate is folded such that, for each of the edge regions, there are multiple folded layers and multiple ones of the folded layers are configured to create a longitudinal dam. FIG. 7A is not necessarily to scale.
**FIG. 7B** is a schematic sectional view of the article of FIG. 7A when its chassis is in a wearable configuration and illustrates the manner in which, for each of the edge regions, multiple ones of the folded layers create a longitudinal dam when the cuffs and free portions of the core lift away from the chassis. FIG. 7B is not necessarily to scale.

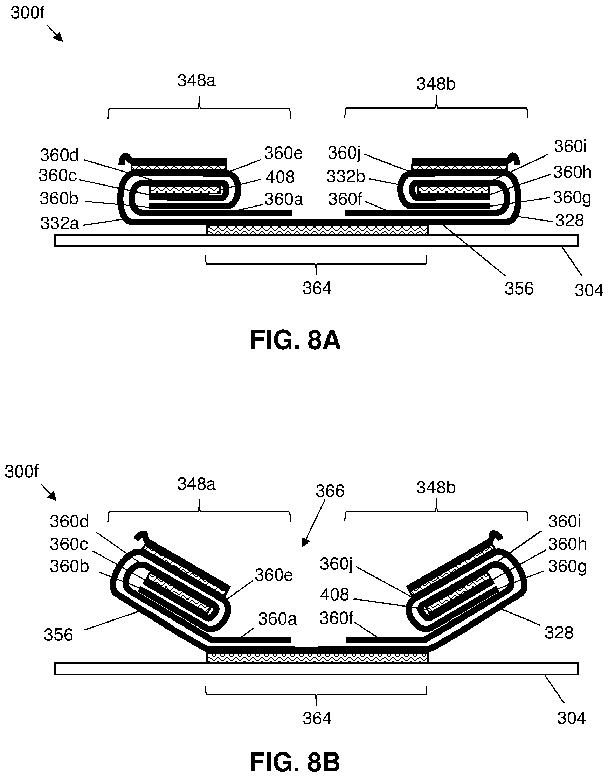

**FIG. 8A** is a schematic sectional view of a sixth embodiment of the present absorbent articles when its chassis is in an extended configuration. The sixth embodiment is substantially similar to the article of FIG. 3A, except that the core includes multiple folded laminates and, for each of the edge regions, at least two of the folded layers are bonded to each other such that the folded structure of the core is at least partially maintained and multiples ones of the folded layers create longitudinal dams when free portions of the core lift away from the chassis. FIG. 8A is not necessarily to scale.
**FIG. 8B** is a schematic sectional view of the article of FIG. 8A when its chassis is in a wearable configuration, illustrating the manner in which, for each of the edge regions, the core's folded structure is at least partially maintained and multiples ones of the folded layers create a longitudinal dam when the cuffs and free portions of the core lift away from the chassis. FIG. 8B is not necessarily to scale.

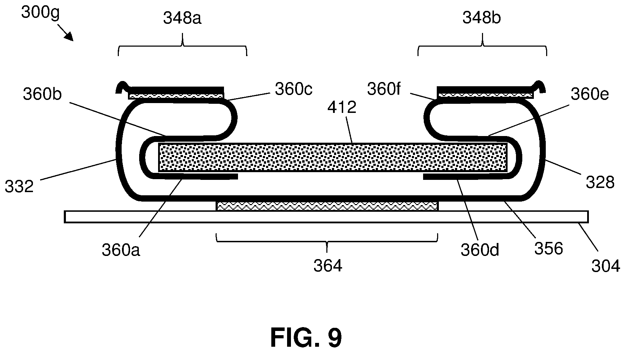

**FIG. 9** is a schematic sectional view of a seventh embodiment of the present absorbent articles when its chassis is in an extended configuration. The seventh embodiment is substantially similar to the article of FIG. 6A, except that a longitudinally-extending insert is coupled to the core such that, for each of the edge regions, a portion of the insert is disposed above the lower layer and below at least one of the folded layers. FIG. 9 is not necessarily to scale.

### DETAILED DESCRIPTION OF ILLUSTRATIVE EMBODIMENTS

Referring to FIGs. 3A-3D, shown is an embodiment 300a of the present absorbent articles. Article 300a can be a baby diaper, training pant, adult incontinence brief or underwear, bladder control pad, feminine hygiene pad, or the like, and comprises a chassis 304. Chassis 304 can have a crotch portion 316 that extends longitudinally between a front portion 308 and a rear portion 312. Chassis 304 can define an extended configuration (FIG. 3A) (e.g., an open configuration if the article is a diaper, training pant, incontinence brief, and the like) and a wearable configuration (e.g., a closed configuration if the article is a diaper, training pant, incontinence brief, and the like) in which crotch portion 316 is configured to conform about the groin area, perineum, and/or rear of a wearer. For example, front portion 308 and rear portion 312 can each include ears and/or fasteners such that the front portion has first and second ends 320a and 320b configured to be coupled to first and second ends 324a and 324b, respectively, of the rear portion. When first ends 320a, 324a and second ends 320b, 324b are so coupled, chassis 304 can defined a wearable configuration in which front and rear portions 308 and 312 can cooperate to encircle and define a waist opening, a left side of chassis 304 can define a first leg opening, and a second side of the chassis can define a second leg opening.

Chassis 304 can have a backsheet configured to face away from a wearer and, optionally, a topsheet configured to face the wearer during use of article 300a. The present backsheets are typically liquid-impermeable and can include, for example, an inner liquid-impermeable film and an outer nonwoven backsheet that can be a nonwoven fabric. A "film" is a membrane-like layer of material formed of one or more polymers, which does not have a form consisting predominately of a web-like structure of fibers and/or other fibers. In some articles, the backsheet can be breathable, for example, an inner liquid-impermeable film of the backsheet can comprise a breathable film. The terms "breathable," "breathable film," "breathable laminate" or "breathable outer cover material" or "breathable backsheet" refers to a film, laminate, or outer cover material having a water vapor transmission rate ("WVTR") of at least about 300 grams/m²/24 hours. Breathable materials typically rely on molecular diffusion of vapor, and are substantially liquid impermeable. "Nonwoven" fabrics, according to an INDA definition, are broadly defined as sheet or web structures bonded together by entangling fiber or filaments, and by perforating films, mechanically, thermally, or chemically. They are flat, porous sheets that are made directly from separate fibers or from molten plastic or plastic film. They are not made by weaving or knitting and do not require converting the fibers to yarn. The basis weight of nonwoven fabrics is usually expressed as gsm or grams per square meter. "Nonwoven backsheet" is a backing substrate layer in the outer cover; a nonwoven backsheet is most often a nonwoven layer facing away from the wearer.

Article 300a can include an absorbent core 328 that extends longitudinally between its opposing first and second ends 392a and 392b. A longitudinal length between first and second ends 392a and 392b can be, for example, greater than or equal to, or between any two of, 300, 330, 360, 390, 420, 450, 480, 510, 540, 570, 560, 600, or more millimeters (mm) (e.g., between 420 and 480 mm). Core 328 is coupled to crotch portion 316, and can, but need not, extend longitudinally along the entire length of the crotch portion. For example, core 328 can have a longitudinal length at least, or between any two of, 10%, 20%, 30%, 40%, 50%, 60%, 70%, 80%, or 90% smaller than a length of crotch portion 316, and optionally can be positioned closer to front portion 308 than to rear portion 312. Core 328 can comprise any material or combination of materials suitable for absorbing liquids, such as, for example, laminate 332. Laminate 332 can have one or more substrate laminae (e.g., 336a and 336b) and one or more absorbent laminae (e.g., 340a).

Each of the absorbent lamina(e) comprises superabsorbent polymer (SAP) particles. "Superabsorbent" or "superabsorbent material" or "SAP" refers to a water-swellable, water-insoluble organic or inorganic material capable, under the most favorable conditions, of absorbing at least about 15 times its weight in an aqueous solution containing 0.9 weight percent sodium chloride and, more desirably, at least about 30 times its weight in an aqueous solution containing 0.9 weight percent sodium chloride and, even more desirably, at least about 50 times its weight in an aqueous solution containing 0.9 weight percent sodium chloride. Exemplary superabsorbent polymer material suitable for use in core 328 can comprise any superabsorbent polymer particles known from superabsorbent literature, for example such as described in Modern Superabsorbent Polymer Technology, F. L. Buchholz, A. T. Graham, Wiley 1998. For example, the SAP particles may be spherical, spherical-like or irregularly shaped particles, such as sausage shaped particles, or ellipsoid shaped particles of the kind typically obtained from inverse phase suspension polymerizations. The SAP particles can also be optionally agglomerated at least to some extent to form larger irregular particles. In some embodiments, the SAP particles can also have a surface modification, such as a partial or full surface coating, for example to increase the hydrophilicity of the SAP particles.

The SAP materials can be natural, synthetic and modified natural polymers and materials. In addition, the SAP materials can be or include organic compounds such as cross linked polymers. "Cross-linked" is a commonly understood term and refers to any approach for effectively rendering normally water-soluble materials substantially water insoluble, but swellable. Such polymers can include, for example, carboxymethylcellulose, alkali metal salts of polyacrylic acids, polyacrylamides, polyvinyl ethers, hydroxypropyl cellulose, polyvinyl morpholinone, polymers and copolymers of vinyl sulfonic acid, polyacrylates, polyacrylamides, polyvinyl pyridine and the like. Other suitable polymers include hydrolyzed acrylonitrile grafted starch, acrylic acid grafted starch, and isobutylene maleic anhydride copolymers, and mixtures thereof. Organic high-absorbency materials can include natural materials, such as agar, pectin, guar gum and peat moss. In addition to organic materials, superabsorbent materials may also include inorganic materials, such as absorbent clays and silica gels. Suitable examples of SAP include T9030, T9600, T9900, and Saviva polymers from BASF Corporation in Charlotte, North Carolina; and W211, W112A, W125, S125D, QX-W1482, QX-W1486, QX-W1504, and QX-W1505 from Nippon Shokubai Co. Ltd, N.A.I.I. in Houston, Texas; and AQUA KEEP SA50 II, SA55SX II, SA60N II, SA65S, HP500E, HP600, and HP 700E from Sumitomo Seika Chemicals Co., Ltd. in Osaka, Japan.

In some articles, the SAP can have a centrifuge retention capacity of 20-60 grams per gram (g/g), for example 30-50 g/g or 33-52 g/g, optionally between 33 and 38 g/g, or optionally between 44 and 48 g/g. The SAP can have particle size distribution (PSD) with most or substantially all particles having a diameter between 150 µm and 850 µm. Preferably, all or substantially all of the SAP particles in at least one of the absorbent lamina(e) have a diameter less than or equal to 500 micrometers (µm) to reduce the roughness of the absorbent lamina. For example, ones of the SAP particles in absorbent lamina 340a having a diameter greater than or equal to 500 µm can account for less than 10% (e.g., less than 3% or less than 0.2%) of the mass of the SAP particles in the lamina. An illustrative SAP suitable for absorbent lamina 340a is HP500E from Sumitomo Seika Chemicals Co., Ltd. in Osaka, Japan. As used herein, particle diameter refers to the equivalent diameter of the particle if the particle is modelled as a sphere.

In some embodiments, the SAP material of the absorbent lamina(e) can be disposed within a matrix of adhesive material. Suitable adhesive material can include, for example, a thermoplastic hot-melt adhesive composition or a pressure-sensitive thermoplastic adhesive composition. For example, absorbent lamina 340a can comprise at least 90% (e.g., greater than 93% or 94%), by weight, SAP and less than or equal to 10% (e.g., less than 6% or 7%), by weight, adhesive. To illustrate, the SAP of absorbent lamina 340a can have a basis weight of at least 40 grams per square meter (gsm), such as, for example, greater than or equal to or between any two of 40, 50, 60, 70, 80, 90, 100 or more gsm (e.g., between 60 and 75 gsm).

Each of the substrate lamina(e) can be constructed from nonwoven material and/or tissue. Suitable nonwoven materials can include, for example, spunbond, spunlace, or carded webs of one or more polymers, including polypropylene, polyethylene, nylon, polyester, and blends of these materials. When constructed from a nonwoven, a substrate lamina can have a basis weight of at least 20 gsm, such as, for example, a basis weight greater than or equal to, or between any two of, 20, 30, 40, 50, 60, 70, 80, 90, 100, 110, 120, 130, or more gsm (e.g., between 20 and 30 gsm, or 28 gsm, or between 45 and 55 gsm). Suitable tissues can include, for example, porous tissues, creped tissues, and standard tissues. When constructed from tissue, a substrate lamina can have a basis weight of at least 10 gsm, such as, for example, a basis weight greater than or equal to, or between any two of, 10, 12, 14, 16, 18, 20, 22, 24, 26, 28, 30, or more gsm.

The substrate lamina(e) can facilitate liquid acquisition and distribution throughout core 328. SAP in the absorbent lamina(e) swells when it absorbs liquid, which can impede liquid distribution throughout core 328 (referred to as "gel blocking"). Tissue and/or nonwoven substrate lamina(e) can mitigate gel blocking by promoting the acquisition and distribution of liquid throughout the absorbent lamina(e). To illustrate, laminate 332 can have a first substrate lamina 336a constructed from tissue, a second substrate lamina 336b constructed from a nonwoven, and an absorbent lamina 340a comprising at least 90% SAP by weight. Absorbent lamina 340a can be in contact with and disposed between first substrate lamina 336a and second substrate lamina 336b (FIG. 3B). First substrate lamina 336a, when constructed from tissue, can provide a capillary network through which liquid is spread and distributed to absorbent lamina 340a, thereby mitigating gel blocking. Second substrate lamina 336b, when constructed from a nonwoven, can absorb and distribute rapid insults of liquid to reduce leakage, and can promote comfort. For example, laminate 332 can be coupled to chassis 304 such that, at least for a portion of the laminate, second substrate lamina 336b is disposed closer to a wearer than are the other lamina(e) when article 300a is worn. Second substrate lamina 336b can thereby function as a topsheet to transfer liquid to absorbent lamina 340a while remaining relatively dry and soft to the touch. Article 300a thus can omit a conventional topsheet that would otherwise cover core 328. In some embodiments, however, core 328 can be disposed between a backsheet and a topsheet of chassis 304.

In other embodiments, laminate 332 can have any suitable number of substrate and absorbent laminae arranged in any suitable order, such as, for example, greater than or equal to or between any two of 1, 2, 3, 4, 5, 6, 7, 8, or more substrate laminae and greater than or equal to or between any two of 1, 2, 3, 4, 5, 6, 7, 8, or more absorbent laminae. For example, any two adjacent laminae in laminate 332 can be the same type of laminae (e.g., both can be substrate laminae or absorbent laminae) or laminae of different types (e.g., one can be one of the substrate lamina(e) and one can be one of the absorbent lamina(e)). By way of illustration, laminate 332 can comprise three substrate laminae and two absorbent laminae arranged such that each of the absorbent laminae is disposed between two of the substrate laminae. Providing additional laminae can increase the absorption capacity of laminate 332.

Laminate 332 can be folded one or more times such that core 328 includes multiple laminate layers, including a lower layer 356 and one or more folded layers (e.g., 360a and 360b) disposed on the lower layer within each of first and second longitudinally-extending edge regions 348a and 348b. For example, as shown, core 328 includes a folded layer 360a in first edge region 348a and a folded layer 360b in second edge region 348b. Lower layer 356 can span lateral width 344 of core 328, and folded layers 360a and 360b can each span less than half of width 344 such that a gap is disposed between the folded layers. For example, each of edge regions 348a and 348b can have a width (e.g., 352a and 352b, respectively) that spans less than or equal to, or between any two of, 50%, 40%, 30%, 20%, or 10% (e.g., between 20% and 46% or between 40% and 46%) of width 344. To illustrate, width 344 of core 328 can be greater than or equal to, or between any two of, 80, 90, 100, 110, 120, 130, 140, or more millimeters (mm) (e.g., between 100 and 120 mm) and each of widths 352a and 352b can be less than or equal to, or between any two of, 70, 60, 50, 40, 30, 20, 10, or fewer millimeters (mm) (e.g., between 10 and 20 mm or between 30 and 65 mm).

Core 328 is coupled to chassis 304 such that the core defines a containment zone 366 for liquids and/or feces when article 300a is worn. Lower layer 356 has a center region 364 coupled to crotch portion 316 via a bond 372, which can be, for example, an adhesive, ultrasonic, or thermal bond. Center region 364 spans a lateral distance 368 that is smaller than width 344, such as, for example, a distance that is smaller than or between any two of 90%, 80%, 70%, 60%, 50%, 40%, 30%, or 20% of width 344. To illustrate, distance 368 can be less than or equal to, or between any two of, 120, 110, 100, 90, 80, 70, 60, 50, 40, 30, 20, 10, or fewer millimeters (mm) (e.g., between 70 and 90 mm). Center region 364 can, but need not, overlap edge regions 348a and 348b. For example, at least one of the folded layers in each of edge regions 348a and 348b can be partially disposed on lower layer 356 within center region 364. Because bond 372 spans only part of width 344, at least a portion of core 328 within each of edge regions 348a and 348b can be free and lift away from chassis 304 towards a wearer when the chassis is in the wearable configuration (FIG. 3D). The liftable portion(s) of core 328 can be configured to lift away from chassis 304 when garment 300a is worn even if the portion(s) are not elasticated. As a result, core 328 can define a zone 366 that promotes liquid and/or feces containment. Liquids and/or feces that escape containment zone 366 can be absorbed from underneath the lifted portions of core 328, further mitigating leakage from article 300a.

Edge regions 348a and 348b, by having multiple layers of laminate 332, can have more SAP than the other portions of laminate 332. The additional SAP can both increase the absorption capacity of the edges of laminate 332 and facilitate formation of containment zone 366 when the SAP in the edge regions swells. And when one of the substrate lamina(e) functions as a topsheet (e.g., when the substrate lamina is a nonwoven), article 300a can omit a conventionally-used topsheet layer that would otherwise impede the expansion and/or unfolding of core 328 in use. For example, a nonwoven substrate lamina can be disposed closer to a wearer than are the other lamina(e) when article 300a is worn, at least for lower layer 336 and additionally, or alternatively, for an uppermost one of the folded layer(s) in each of edge regions 348a and 348b.

Article 300a can include cuffs 376 coupled to at least one of the folded layers to facilitate the lifting of the free portions of core 328. For example, within each of edge regions 348a and 348b, a cuff 376 can be coupled to an uppermost one of the folded layer(s) (e.g., 360a or 360b) via a bond 388, such as, for example, an adhesive, ultrasonic, or thermal bond. In other embodiments, however, each of cuffs 376 can be bonded to a respective one of the outermost, longitudinally-extending folded edges of core 328. Cuffs 376 can be configured to lift away from chassis 304 when the chassis is in the wearable configuration and thereby urge the free portions of core 328 to unfold away from the chassis as well. For example, as shown, each of cuffs 376 has opposing first and second longitudinally-extending sides 380 and 384 and is bonded to a respective one of folded layers 360a and 360b such that the first side is disposed closer to the other of the folded layers than is the second side. For each of cuffs 376, the cuff can be elastic and/or second side 384 can be ruffled such that, when chassis 304 is in the wearable configuration, the second side rises (e.g., such that the second side is further from the chassis than is first side 380) and thereby urges at least a portion of core 328 away from the chassis. When so bonded, cuffs 376 can also at least partially unfold the core to increase the volume of containment zone 366. The free portions of core 328 and each of cuffs 376, when lifted, can form a leak-prevention gasket around the legs of a wearer to further mitigate leakage.

Additionally, or alternatively, laminate 332 can be riffled such that a plurality of ridges extend longitudinally (e.g., in the machine direction) along the laminate. The ridges can also promote the lifting of the free portions of core 328 away from chassis 304.

Article 300a can include end caps 396. Each of end caps 396 can be coupled to chassis 304, extend laterally across a respective one of first and seconds ends 392a and 392b of core 328, and, optionally, extend longitudinally to a respective one of the front and rear edges of chassis 300a. End caps 396 can inhibit leakage out of ends 392a and 392b while still permitting portions of core 328 to lift away from chassis 304.

As shown, cuffs 376 are not connected to chassis 304, but can be connected to the chassis in other embodiments. Referring to FIGs. 4A and 4B, shown is an article 300b which is substantially similar to article 300a, the primary exception being the configuration of cuffs 376. As shown, each of cuffs 376 has first and second portions 400 and 404. First portion 400 can be coupled to chassis 304 (e.g., on crotch portion 316) and second portion 404 can be bonded to at least one of the folded layers in a respective one of edge regions 348a and 348b (e.g., to the uppermost folded layer). In this configuration, second side 384 (e.g., a ruffled side) of each of cuffs 376 can be disposed closer to the other of the edge regions than is first side 380. When chassis 304 is in the wearable configuration, second portion 404 can lift and thereby urge free portions of core 328 away from the chassis to define containment zone 366. For example, each of cuffs 376 can lift at least one of the folded layer(s) in each of edge regions 348a and 348b. Cuffs 376 of article 300b may lift portions of lower layer 356 away from chassis 304, but to a lesser extent than those of article 300a.

Core 328 can include any suitable number of folded layers within each of edge regions 348a and 348b, such as, for example, greater than or equal to or between any two of 1, 2, 3, 4, 5, 6, 7, 8, or more folded layers. For example, referring to FIGs. 5A and 5B, shown is an article 300c which is substantially similar to article 300a, the primary exception being that article 300c has four folded layers: two (360a-360c) within first edge region 348a and two (360c-360d) within second edge region 348b. Providing multiple folded layers in each of edge regions 348a and 348b can improve liquid containment and increase the absorption capacity of core 328 at least because the additional folded layers can increase the amount of SAP in the edge regions. As a result, containment zone 366 can be defined by barriers having a higher absorption capacity such that the containment zone can hold more liquid and/or feces.

Some cores can be folded such that one or more of the folded layer(s) in each of edge regions 348a and 348b form longitudinal dams to improve liquid containment. For example, referring to FIGs. 6A and 6B, shown is an article 300d which is substantially similar to article 300a, the primary exception being the folded structure of core 328. As shown, article 300d can have six folded layers: three (360a-360c) within first edge region 348a and three (360d-360f) within second edge region 348b. Laminate 332 can be folded such that each of the lowermost folded layers (e.g., 360a and 360e) extends inwardly from a fold of the laminate to an end of the laminate (e.g., 374a and 374b, respectively), the end disposed closer to the other of the edge regions than is the fold. When the free portions of core 328 are lifted, folded layers 360a and 360e can remain closer to lower layer 356 than are the other folded layers. For example, at least a portion of each of folded layers 360a and 360e can remain disposed on lower layer 356 and, when they swell, create longitudinal dams.

In some cores, longitudinal dams can be formed from multiple folded layers. Referring to FIGs. 7A and 7B, shown is an article 300e which is substantially similar to article 300a, with the primary exception being the folded structure of core 328. As shown, article 300e can have ten folded layers: five (360a-360e) within first edge region 348a and five (360f-360j) within second edge region 348b. Within each of edge regions 348a and 348b, core 328 can have a folded layer (e.g., 360b and 360g, respectively) that extends inwardly from a fold of the laminate to an end of the laminate (e.g., 374a and 374b, respectively), the end disposed closer to the other of the edge regions than is the fold. Inwardly-extending folded layers 360b and 360g can each be disposed between two other of the folded layers (e.g., folded layer 360b can be layered between folded layers 360a and 360c, and folded layer 360g can be layered between folded layers 360f and 360h). As a result, the folded structure of core 328 can at least partially be maintained such that two or more of the folded layers within each of edge regions 348a and 348b (e.g., those closest to lower layer 156, such as 360a-360c and 360f-360h, respectively) can remain at least partially in contact with each other and/or the lowermost folded layer can remain on the lower layer when the free portions of the core are lifted. These folded layers (e.g., 360a-360c and 360f-360h) can create longitudinal dams within each of edge regions 348a and 348b that have a higher absorption capacity and larger swellable thickness to improve liquid containment.

Referring to FIGs. 8A, and 8B, shown is another article 300f which is substantially similar to article 300a, with the primary exception being the folded structure of core 328. As shown, article 300f can have ten folded layers: five (360a-360e) within edge region 348a and five (360f-360j) within edge region 348b. In each of edge regions 348a and 348b, at least one folded layer (e.g., 360c or 360h) can be bonded to at least one other folded layer (e.g., 360d or 360i, respectively) via bond 408, which can be an adhesive, ultrasonic, or adhesive bond. Bond 408 can at least partially maintain the folded structure of core 328 when the free portions of the core are lifted to create longitudinal dams that each have multiple folded layers.

Additionally, or alternatively, core 328 can comprise multiple laminates 332a and 332b, each having one of the arrangements of absorbent lamina(e) and substrate lamina(e) described above with respect to laminate 332. Laminates 332a and 332b can, but need not, have the same arrangement of laminae. As shown, laminates 332a and 332b can be layered such that lower layer 356 and folded layers 360b, 360e, 360g, and 360j originate from laminate 332a and folded layers 360a, 360c-360d, 360f, and 360h-360i originate from laminate 332b. In other embodiments, core 328 can have any suitable number of laminates, such as, for example, greater than or equal to or between any two of 1, 2, 3, 4, 5, 6, 7, 8, or more laminates layered and folded such that lower layer 356 originates from one of the laminate(s), and greater than or equal to, or between any two of, 1, 2, 3, 4, 5, 6, 7, 8, or more folded layers originate from each of the laminate(s).

The folded layers can, but need not, be the same size, regardless of whether core 328 comprises a single laminate (e.g., 332) or multiple laminates (e.g., 332a and 332b). To illustrate, for each of edge regions 348a and 348b, each of the folded layer(s) can span the entire width of the edge region (e.g., 352a or 352b) but, in some embodiments, one or more of the folded layer(s) can span a distance that is less than, or between any two of, 10%, 20%, 30%, 40%, 50%, 60%, 70%, 80%, or 90% of the width of the edge region. For example, as shown, folded layers 360a and 360f can each span a lateral distance larger than that spanned by each of the other folded layers. When core 328 has differently-sized folded layers, the core can define multiple channels between edge regions 348a and 348b (e.g., one between folded layers 360a and 360f and one between the stack of folded layers 360b-360e and the stack of folded layers 360g-360j). Providing multiple channels can improve fluid management.

Referring to FIG. 9, shown is another article 300g which is substantially similar to article 300d, the primary exception being the inclusion of a longitudinally-extending insert 412 coupled to laminate 332. Insert 412 can comprise fluff and SAP to increase the absorption capacity of core 328 and additionally, or alternatively, can comprise a through-air bonded polymer nonwoven. When constructed from a through-air bonded polymer nonwoven, insert 412 can absorb and distribute rapid insults of liquid throughout laminate 332.

Within each of edge regions 348a and 348b, a portion of insert 412 can be disposed above lower layer 156 and below at least one of the folded layer(s) (e.g., 360b-360c and 360e-360f) to mitigate leakage and promote insert retention. For example, liquid absorbed and distributed by insert 412 can be contained within containment zone 366 to reduce runoff. As shown, a first portion of insert 412 is disposed between folded layers 360a and 360b and a second portion of the insert is disposed between folded layers 360d and 360e. In other embodiments, however, insert 412 can be disposed on lower layer 156 such that, within each of edge regions 348a and 348b, a portion of the insert is disposed between the lower layer and one of the folded layers (e.g., 360a or 360d).

In some embodiments, core 328 can be disposed above insert 412 (e.g., when the insert comprises fluff and SAP). For example, insert 412 can be integrated into chassis 304 (e.g., between a topsheet and a backsheet of the chassis) and lower layer 156 can be bonded to the topsheet. Additionally or alternatively, lower layer 156 can be bonded to an upper surface of core 328. The materials used in laminate 332 of core 328 can be selected such that the core has comparatively higher permeability to facilitate liquid transfer to insert 412 and maintain a dry wearer-facing surface. For example, second substrate lamina 336b can comprise a comparatively lighter nonwoven (e.g., having a basis weight between 20 and 35 gsm) and absorbent lamina 340a can comprise a comparatively lighter SAP (e.g., having a basis weight between 60 and 75 gsm).

FIGs. 3B-3D, 4A-4B, 5A-5B, 6A-6B, 7A-7B, 8A-8B, and 9 are exaggerated to better understand the overall structure of the present articles (e.g., 300a-300g), laminates (e.g., 332), and absorbent cores (e.g., 328) and, as such, are for illustrative purpose only and are not necessarily to scale. For example, the figures illustrate the relative positions and relationships between elements of the present articles, including, for example, the position of laminae in a laminate (e.g., 332), the general folded structure of a core (e.g., 328), and the manner in which free portions of the core lift away from a chassis (e.g., 304) to define a containment zone (e.g., 366), and should not be interpreted to limit the invention. More particularly, while the schematic views illustrate gaps between adjacent laminate layers, in application the core has a more compact thickness and adjacent laminate layers can be in contact with each other. The present laminates can have a thickness, for example, greater than or equal to, or between any two of, 0.20, 0.25, 0.30, 0.35, 0.40, 0.45, 0.50, 0.55, 0.60, 0.65, 0.70, or more millimeters (mm) (e.g., between 0.40 and 0.50 mm). And while some of the schematic views represent the present laminates as a single black line, it should understood that the depicted laminates comprise any of the above-described arrangements of substrate lamina(e) (e.g., 336a and 336b) and absorbent lamina(e) (e.g., 340a) and can have any suitable orientation relative to the chassis.

The above specification and examples provide a complete description of the structure and use of illustrative embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the methods and systems are not intended to be limited to the particular forms disclosed. Rather, they include all modifications and alternatives falling within the scope of the claims, and embodiments other than the one shown may include some or all of the features of the depicted embodiment. For example, elements may be omitted or combined as a unitary structure, and/or connections may be substituted. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several embodiments.

The claims are not intended to include, and should not be interpreted to include, means-plus- or step-plus-function limitations, unless such a limitation is explicitly recited in a given claim using the phrase(s) "means for" or "step for," respectively.

## Claims

1. An absorbent article (300a, 300b, 300c, 300d, 300e, 300f, 300g) comprising:
a chassis (304) having opposing front and rear portions (308, 312) and a crotch portion (316) extending longitudinally between the front and rear portions (308, 312), wherein the crotch portion (316) is configured to conform about at least one of a wearer's groin area, perineum, and rear when the chassis (304) is in a wearable configuration;
an absorbent core (328) extending longitudinally along the crotch portion (316) and comprising a laminate (332) that includes two or more substrate laminae and one or more absorbent laminae (340), wherein:
each of the absorbent lamina(e) (340) comprises superabsorbent polymer (SAP);
a first one of the absorbent lamina(e) (340) is disposed between first and second ones of the substrate laminae (340); and
the laminate (332) is longitudinally folded such that the absorbent core (328) includes:
a lower layer (356) of the laminate (332) spanning a lateral width (344) of the absorbent core (328); and
one or more folded layers (360a, 360b, 360c, 360d, 360e, 360f, 360g, 360h, 360i, 360j) of the laminate (332) disposed above the lower layer (356) within each of first and second longitudinally-extending edge regions (348a, 348b), each of the edge regions (348a, 348b) spanning less than 50% of the width (344) of the absorbent core (328);
wherein a center region (364) of the lower layer (356) is bonded to the crotch portion (316), the center region (364) spanning a lateral distance (368) smaller than said lateral width (344) of the absorbent core (328) and at least a portion of the absorbent core (328) in each of the edge regions (348a, 348b) is not bonded to the chassis (304) and thus can lift away from the chassis (304) to define a leakage-mitigating containment zone (366), when the chassis (304) is in the wearable configuration;
wherein the lifted portions provide an absorbent barrier impeding the escape of liquid and/or feces, and the liquid and/or feces escaping the containment zone (366) is absorbed from underneath the lifted portions of the core (328).

2. The absorbent article (300a, 300b, 300c, 300d, 300e, 300f, 300g) of claim 1, comprising first and second longitudinally-extending cuffs (376), each having:
a first portion (400) coupled to the crotch portion (316); and
a second portion (404) bonded to at least one of the folded layer(s) (360a, 360b, 360c, 360d, 360e, 360f, 360g, 360h, 360i, 360j) of a respective one of the first and second edge regions (348a, 348b);
wherein the second portion (404) is configured to lift away from the chassis (304) when the chassis (304) is in the wearable configuration such that the cuff (376) urges at least a portion of the absorbent core (328) away from the chassis (304).

3. The absorbent article (300a, 300b, 300c, 300d, 300e, 300f, 300g) of claim 1, **characterized in that** it comprised first and second longitudinally-extending cuffs (376), wherein
each of the cuffs (376) is bonded to an uppermost one of the folded layer(s) (360a, 360b, 360c, 360d, 360e, 360f, 360g, 360h, 360i, 360j) of a respective one of the first and second edge regions (348a, 348b),
a first side (380) of the each of the cuffs (376) is disposed closer to the other of the edge regions (348a, 348b) than is a second side (384) of the cuff (376); and
the second side (384) is configured to lift away from the chassis (304) when the chassis (304) is in the wearable configuration such that:
the second side (384) is further from the chassis (304) than is the first side (380); and
the cuff (376) urges at least a portion of the absorbent core (328) away from the chassis (304).

4. The absorbent article (300a, 300b, 300c, 300d, 300e, 300f, 300g) of any of claims 1-3, wherein:
the absorbent core (328) has first and second ends and the absorbent core (328) extends longitudinally between the first and second ends; and
for each of the first and second ends, an end cap (396) is coupled to the chassis (304) and extends laterally across the first or second end, respectively.

5. The absorbent article (300a, 300b, 300c, 300d, 300e, 300f, 300g) of any of claims 1-4, wherein the laminate (332) is folded such that for each of the first and second edge regions (348a, 348b) the one or more folded layers (360a, 360b, 360c, 360d, 360e, 360f, 360g, 360h, 360i, 360j) comprise two or more folded layers (360a, 360b, 360c, 360d, 360e, 360f, 360g, 360h, 360i, 360j).

6. The absorbent article (300a, 300b, 300c, 300d, 300e, 300f, 300g) of claim 5, wherein the laminate (332) is folded such that for each of the first and second edge regions (348a, 348b), one of the folded layers (360a, 360b, 360c, 360d, 360e, 360f, 360g, 360h, 360i, 360j):
extends inwardly from a fold of the laminate (332) to an end of the laminate (374a, 374b) such that the end is disposed closer to the other of the edge regions (348a, 348b) than is the fold; and
is disposed below at least one other of the folded layers (360a, 360b, 360c, 360d, 360e, 360f, 360g, 360h, 360i, 360j).

7. The absorbent article (300a, 300b, 300c, 300d, 300e, 300f, 300g) of claim 5, wherein for each of the first and second edge regions (348a, 348b), a first one of the folded layers (360a, 360b, 360c, 360d, 360e, 360f, 360g, 360h, 360i, 360j) is bonded to a second one of the folded layers (360a, 360b, 360c, 360d, 360e, 360f, 360g, 360h, 360i, 360j) with an adhesive.

8. The absorbent article (300a, 300b, 300c, 300d, 300e, 300f, 300g) of any of claims 1-7, wherein the first substrate lamina comprises tissue.

9. The absorbent article (300a, 300b, 300c, 300d, 300e, 300f, 300g) of claim 8, wherein the second substrate lamina comprises a nonwoven and, for the lower layer (356), the second substrate lamina is configured to be disposed closer to a wearer than is the first substrate lamina when the absorbent article is worn.

10. The absorbent article (300a, 300b, 300c, 300d, 300e, 300f, 300g) of any of claims 1-9, wherein the laminate (332) is folded such that, for each of the first and second edge regions (348a, 348b):
a lowermost one of the folded layer(s) (360a, 360b, 360c, 360d, 360e, 360f, 360g, 360h, 360i, 360j) has and extends laterally between first and second portions, the first portion disposed closer to the other of the edge regions (348a, 348b) than is the second portion; and
the first portion is configured to be disposed on the lower layer (356) when the chassis (304) is in the wearable configuration.

11. The absorbent article (300a, 300b, 300c, 300d, 300e, 300f, 300g) of any of claims 1-10, wherein the SAP of each of the absorbent lamina(e) (340) has a centrifuge retention capacity between 33 and 52 grams per gram (g/g).

12. The absorbent article (300a, 300b, 300c, 300d, 300e, 300f, 300g) of any of claims 1-11, wherein the SAP of each of the absorbent lamina(e) (340) has a basis weight between 60 and 75 grams per square meter (gsm).

13. The absorbent article (300a, 300b, 300c, 300d, 300e, 300f, 300g) of any of claims 1-12, comprising:
a longitudinally-extending insert (412) coupled to the laminate (332) such that for each of the first and second edge regions (348a, 348b) a portion of the insert (412) is disposed above the lower layer (356) and below at least one of the folded layer(s) (360a, 360b, 360c, 360d, 360e, 360f, 360g, 360h, 360i, 360j);
wherein the insert (412) comprises at least one of:
fluff and SAP; and
a through-air bonded polymer nonwoven.

14. The absorbent article (300a, 300b, 300c, 300d, 300e, 300f, 300g) of any of claims 1-13, wherein:
the two or more substrate laminae comprise three or more substrate laminae;
the one or more absorbent laminae (340) comprise two or more absorbent laminae (340); and
a second one of the absorbent laminae (340) is disposed between the second substrate lamina and a third one of the substrate laminae.

15. The absorbent article (300a, 300b, 300c, 300d, 300e, 300f, 300g) of any of claims 1-14, wherein each of the first and second edge regions (348a, 348b) spans at least 20% of the width (344) of the absorbent core (328).

## Patentansprüche

1. Ein absorbierender Artikel (300a, 300b, 300c, 300d, 300e, 300f, 300g), aufweisend:
ein Chassis (304) mit gegenüberliegenden vorderen und hinteren Abschnitten (308, 312) und einem Schrittabschnitt (316), der sich in Längsrichtung zwischen den vorderen und hinteren Abschnitten (308, 312) erstreckt, wobei der Schrittabschnitt (316) so konfiguriert ist, dass er sich um mindestens einen der folgenden Bereiche anpasst: Leistenbereich, Beckenboden und Gesäß eines Trägers, wenn sich das Chassis (304) in einer tragbaren Konfiguration befindet;
einen absorbierenden Kern (328), der sich in Längsrichtung entlang des Schrittabschnitts (316) erstreckt und ein Laminat (332) aufweist, das zwei oder mehr Substratlaminae und eine oder mehrere absorbierende Laminae (340) aufweist, wobei:
jede der absorbierenden Lamina(e) (340) ein superabsorbierendes Polymer (SAP) aufweist;
eine erste der absorbierenden Lamina(e) (340) zwischen den ersten und zweiten Substratlaminae (340) angeordnet ist; und
das Laminat (332) in Längsrichtung gefaltet ist, so dass der absorbierende Kern (328) aufweist:
eine untere Lage (356) des Laminats (332), die eine seitliche Breite (344) des absorbierenden Kerns (328) überspannt; und
eine oder mehrere gefaltete Lagen (360a, 360b, 360c, 360d, 360e, 360f, 360g, 360h, 360i, 360j) des Laminats (332), die über der unteren Lage (356) innerhalb jedem der ersten und zweiten, sich in Längsrichtung erstreckenden Randbereiche (348a, 348b) angeordnet sind, wobei jeder der Randbereiche (348a, 348b) weniger als 50 % der Breite (344) des absorbierenden Kerns (328) überspannt;
wobei ein Mittelbereich (364) der unteren Lage (356) mit dem Schrittabschnitt (316) verbunden ist, der Mittelbereich (364) eine seitliche Distanz (368) überspannt, die kleiner ist als die seitliche Breite (344) des absorbierenden Kerns (328), und mindestens ein Abschnitt des absorbierenden Kerns (328) in jedem der Randbereiche (348a, 348b) nicht mit dem Chassis (304) verbunden ist und sich somit vom Chassis (304) abheben kann, um eine auslaufminimierende Rückhaltezone (366) zu definieren, wenn sich das Chassis (304) in der tragbaren Konfiguration befindet;
wobei die abgehobenen Abschnitte eine absorbierende Barriere bereitstellen, die das Austreten von Flüssigkeit und/oder Fäkalien hemmt, und die Flüssigkeit und/oder Fäkalien, welche aus der Rückhaltezone (366) austreten, von unterhalb der abgehobenen Abschnitte des Kerns (328) absorbiert werden.

2. Der absorbierende Artikel (300a, 300b, 300c, 300d, 300e, 300f, 300g) nach Anspruch 1, aufweisend erste und zweite, sich in Längsrichtung erstreckende Bündchen (376), die jeweils aufweisen:
einen ersten Abschnitt (400), der mit dem Schrittabschnitt (316) gekoppelt ist; und
einen zweiten Abschnitt (404), der mit mindestens einer der gefalteten Lage(n) (360a, 360b, 360c, 360d, 360e, 360f, 360g, 360h, 360i, 360j) eines jeweiligen der ersten und zweiten Randbereiche (348a, 348b) verbunden ist;
wobei der zweite Abschnitt (404) so konfiguriert ist, dass er sich vom Chassis (304) abhebt, wenn sich das Chassis (304) in der tragbaren Konfiguration befindet, so dass das Bündchen (376) mindestens einen Abschnitt des absorbierenden Kerns (328) vom Chassis (304) wegdrückt.

3. Der absorbierende Artikel (300a, 300b, 300c, 300d, 300e, 300f, 300g) nach Anspruch 1, **dadurch gekennzeichnet, dass** er erste und zweite, sich in Längsrichtung erstreckende Bündchen (376) aufweist, wobei
jedes der Bündchen (376) mit einer obersten der gefalteten Lage(n) (360a, 360b, 360c, 360d, 360e, 360f, 360g, 360h, 360i, 360j) eines jeweiligen der ersten und zweiten Randbereiche (348a, 348b) verbunden ist,
eine erste Seite (380) von jedem der Bündchen (376) näher an dem anderen der Randbereiche (348a, 348b) angeordnet ist als eine zweite Seite (384) des Bündchens (376); und
die zweite Seite (384) so konfiguriert ist, dass sie sich vom Chassis (304) abhebt, wenn sich das Chassis (304) in der tragbaren Konfiguration befindet, so dass:
die zweite Seite (384) weiter vom Chassis (304) entfernt ist als die erste Seite (380); und
das Bündchen (376) mindestens einen Abschnitt des absorbierenden Kerns (328) vom Chassis (304) wegdrückt.

4. Der absorbierende Artikel (300a, 300b, 300c, 300d, 300e, 300f, 300g) nach einem der Ansprüche 1 - 3, wobei:
der absorbierende Kern (328) erste und zweite Enden hat und der absorbierende Kern (328) sich in Längsrichtung zwischen den ersten und zweiten Enden erstreckt; und
für jedes der ersten und zweiten Enden eine Endkappe (396) mit dem Chassis (304) gekoppelt ist und sich seitlich über das erste bzw. zweite Ende erstreckt.

5. Der absorbierende Artikel (300a, 300b, 300c, 300d, 300e, 300f, 300g) nach einem der Ansprüche 1 - 4, wobei das Laminat (332) so gefaltet ist, dass für jeden der ersten und zweiten Randbereiche (348a, 348b) die eine oder mehreren gefalteten Lagen (360a, 360b, 360c, 360d, 360e, 360f, 360g, 360h, 360i, 360j) zwei oder mehr gefaltete Lagen (360a, 360b, 360c, 360d, 360e, 360f, 360g, 360h, 360i, 360j) aufweisen.

6. Der absorbierende Artikel (300a, 300b, 300c, 300d, 300e, 300f, 300g) nach Anspruch 5, wobei das Laminat (332) so gefaltet ist, dass für jeden der ersten und zweiten Randbereiche (348a, 348b) eine der gefalteten Lagen (360a, 360b, 360c, 360d, 360e, 360f, 360g, 360h, 360i, 360j):
sich von einer Faltung des Laminats (332) nach innen zu einem Ende des Laminats (374a, 374b) erstreckt, so dass das Ende näher an dem anderen der Randbereiche (348a, 348b) angeordnet ist als die Faltung; und
unterhalb von mindestens einer anderen der gefalteten Lagen (360a, 360b, 360c, 360d, 360e, 360f, 360g, 360h, 360i, 360j) angeordnet ist.

7. Der absorbierende Artikel (300a, 300b, 300c, 300d, 300e, 300f, 300g) nach Anspruch 5, wobei für jeden der ersten und zweiten Randbereiche (348a, 348b) eine erste der gefalteten Lagen (360a, 360b, 360c, 360d, 360e, 360f, 360g, 360h, 360i, 360j) mit einem Klebstoff an eine zweite der gefalteten Lagen (360a, 360b, 360c, 360d, 360e, 360f, 360g, 360h, 360i, 360j) verbunden ist.

8. Der absorbierende Artikel (300a, 300b, 300c, 300d, 300e, 300f, 300g) nach einem der Ansprüche 1 - 7, wobei die erste Substratlamina Gewebe aufweist.

9. Der absorbierende Artikel (300a, 300b, 300c, 300d, 300e, 300f, 300g) nach Anspruch 8, wobei die zweite Substratlamina ein Vlies aufweist, und, für die untere Lage (356), die zweite Substratlamina so konfiguriert ist, dass sie beim Tragen des absorbierenden Artikels näher an einem Träger angeordnet ist als die erste Substratlamina.

10. Der absorbierende Artikel (300a, 300b, 300c, 300d, 300e, 300f, 300g) nach einem der Ansprüche 1 - 9, wobei das Laminat (332) so gefaltet ist, dass für jeden der ersten und zweiten Randbereiche (348a, 348b):
eine unterste der gefalteten Lage(n) (360a, 360b, 360c, 360d, 360e, 360f, 360g, 360h, 360i, 360j) erste und zweite Abschnitte hat und sich seitlich zwischen diesen erstreckt, wobei der erste Abschnitt näher an dem anderen der Randbereiche (348a, 348b) angeordnet ist als der zweite Abschnitt; und
der erste Abschnitt so konfiguriert ist, dass er auf der unteren Lage (356) angeordnet ist, wenn sich das Chassis (304) in der tragbaren Konfiguration befindet.

11. Der absorbierende Artikel (300a, 300b, 300c, 300d, 300e, 300f, 300g) nach einem der Ansprüche 1 - 10, wobei das SAP von jeder der absorbierenden Lamina(e) (340) eine Zentrifugenrückhaltekapazität zwischen 33 und 52 Gramm pro Gramm (g/g) aufweist.

12. Der absorbierende Artikel (300a, 300b, 300c, 300d, 300e, 300f, 300g) nach einem der Ansprüche 1 - 11, wobei das SAP jeder der absorbierenden Lamina(e) (340) ein Flächengewicht zwischen 60 und 75 Gramm pro Quadratmeter (g/m²) aufweist.

13. Der absorbierende Artikel (300a, 300b, 300c, 300d, 300e, 300f, 300g) nach einem der Ansprüche 1 - 12, aufweisend:
einen sich in Längsrichtung erstreckenden Einsatz (412), der mit dem Laminat (332) so gekoppelt ist, dass für jeden der ersten und zweiten Randbereiche (348a, 348b) ein Abschnitt des Einsatzes (412) über der unteren Lage (356) und unter mindestens einer der gefalteten Lage(n) (360a, 360b, 360c, 360d, 360e, 360f, 360g, 360h, 360i, 360j) angeordnet ist;
wobei der Einsatz (412) mindestens eines der Folgenden aufweist:
Flaum und SAP; und
ein durch "Trough-Air Bonding" verfestigtes Polymervlies.

14. Der absorbierende Artikel (300a, 300b, 300c, 300d, 300e, 300f, 300g) nach einem der Ansprüche 1 - 13, wobei:
die zwei oder mehr Substratlaminae drei oder mehr Substratlaminae aufweisen;
die eine oder mehreren absorbierenden Laminae (340) zwei oder mehrere absorbierende Laminae (340) aufweisen; und
eine zweite der absorbierenden Laminae (340) zwischen der zweiten Substratlamina und einer dritten der Substratlaminae angeordnet ist.

15. Der absorbierende Artikel (300a, 300b, 300c, 300d, 300e, 300f, 300g) nach einem der Ansprüche 1 - 14, wobei jeder der ersten und zweiten Randbereiche (348a, 348b) mindestens 20 % der Breite (344) des absorbierenden Kerns (328) überspannt.

## Revendications

1. Article absorbant (300a, 300b, 300c, 300d, 300e, 300f, 300g) comprenant :
un châssis (304) ayant des portions avant et arrière (308, 312) opposées et une portion d'entrejambe (316) s'étendant longitudinalement entre les portions avant et arrière (308, 312), dans lequel la portion d'entrejambe (316) est configurée pour se conformer à au moins une parmi une zone de l'aine, le périnée et le derrière d'un porteur lorsque le châssis (304) est dans une configuration portable ;
une âme absorbante (328) s'étendant longitudinalement le long de la portion d'entrejambe (316) et comprenant un stratifié (332) qui inclut deux feuillets de substrat ou plus et un ou plusieurs feuillets absorbants (340), dans lequel :
chacun des feuillets absorbants (340) comprend un polymère superabsorbant (SAP) ;
un premier feuillet des feuillets absorbants (340) est disposé entre des premier et deuxième feuillets des feuillets de substrat (340) ; et
le stratifié (332) est plié longitudinalement de telle sorte que l'âme absorbante (328) inclut :
une couche inférieure (356) du stratifié (332) couvrant une largeur latérale (344) de l'âme absorbante (328) ; et
une ou plusieurs couches pliées (360a, 360b, 360c, 360d, 360e, 360f, 360g, 360h, 360i, 360j) du stratifié (332) disposées au-dessus de la couche inférieure (356) dans chacune de première et seconde régions de bord (348a, 348b) s'étendant longitudinalement, chacune des régions de bord (348a, 348b) couvrant moins de 50 % de la largeur (344) de l'âme absorbante (328) ;
dans lequel une région centrale (364) de la couche inférieure (356) est liée à la portion d'entrejambe (316), la région centrale (364) couvrant une distance latérale (368) plus petite que ladite largeur latérale (344) de l'âme absorbante (328) et au moins une portion de l'âme absorbante (328) dans chacune des régions de bord (348a, 348b) n'est pas liée au châssis (304) et peut donc se soulever loin du châssis (304) pour définir une zone de confinement (366) atténuant les fuites, lorsque le châssis (304) est dans la configuration portable ;
dans lequel les portions soulevées constituent une barrière absorbante empêchant l'échappement de liquide et/ou de matières fécales, et le liquide et/ou les matières fécales s'échappant de la zone de confinement (366) sont absorbés par le dessous des portions soulevées de l'âme (328).

2. Article absorbant (300a, 300b, 300c, 300d, 300e, 300f, 300g) selon la revendication 1, comprenant des premier et second revers (376) s'étendant longitudinalement, chacun ayant :
une première portion (400) couplée à la portion d'entrejambe (316) ; et
une seconde portion (404) liée à au moins une des couches pliées (360a, 360b, 360c, 360d, 360e, 360f, 360g, 360h, 360i, 360j) d'une région respective des première et seconde régions de bord (348a, 348b) ;
dans lequel la seconde portion (404) est configurée pour se soulever loin du châssis (304) lorsque le châssis (304) est dans la configuration portable de telle sorte que le revers (376) pousse au moins une portion de l'âme absorbante (328) loin du châssis (304).

3. Article absorbant (300a, 300b, 300c, 300d, 300e, 300f, 300g) selon la revendication 1, **caractérisé en ce qu'**il comprend des premier et second revers (376) s'étendant longitudinalement, dans lequel
chacun des revers (376) est lié à une couche la plus supérieure des couches pliées (360a, 360b, 360c, 360d, 360e, 360f, 360g, 360h, 360i, 360j) d'une région respective des premières et secondes régions de bord (348a, 348b),
un premier côté (380) de chacun des revers (376) est disposé plus près de l'autre des régions de bord (348a, 348b) que ne l'est un second côté (384) du revers (376) ; et
le second côté (384) est configuré pour se soulever loin du châssis (304) lorsque le châssis (304) est dans la configuration portable de telle sorte que :
le second côté (384) est plus éloigné du châssis (304) que ne l'est le premier côté (380) ; et
le revers (376) pousse au moins une portion de l'âme absorbante (328) loin du châssis (304).

4. Article absorbant (300a, 300b, 300c, 300d, 300e, 300f, 300g) selon l'une quelconque des revendications 1 à 3, dans lequel :
l'âme absorbante (328) a des première et seconde extrémités et l'âme absorbante (328) s'étend longitudinalement entre les première et seconde extrémités ; et
pour chacune des première et seconde extrémités, un capuchon d'extrémité (396) est couplé au châssis (304) et s'étend latéralement à travers la première ou la seconde extrémité, respectivement.

5. Article absorbant (300a, 300b, 300c, 300d, 300e, 300f, 300g) selon l'une quelconque des revendications 1 à 4, dans lequel le stratifié (332) est plié de telle sorte que pour chacune des première et seconde régions de bord (348a, 348b), les une ou plusieurs couches pliées (360a, 360b, 360c, 360d, 360e, 360f, 360g, 360h, 360i, 360j) comprennent deux couches pliées (360a, 360b, 360c, 360d, 360e, 360f, 360g, 360h, 360i, 360j) ou plus.

6. Article absorbant (300a, 300b, 300c, 300d, 300e, 300f, 300g) selon la revendication 5, dans lequel le stratifié (332) est plié de telle sorte que pour chacune des première et seconde régions de bord (348a, 348b), l'une des couches pliées (360a, 360b, 360c, 360d, 360e, 360f, 360g, 360h, 360i, 360j) :
s'étend vers l'intérieur à partir d'un pli du stratifié (332) jusqu'à une extrémité du stratifié (374a, 374b) de telle sorte que l'extrémité est disposée plus près de l'autre des régions de bord (348a, 348b) que ne l'est le pli ; et
est disposée sous au moins une autre des couches pliées (360a, 360b, 360c, 360d, 360e, 360f, 360g, 360h, 360i, 360j).

7. Article absorbant (300a, 300b, 300c, 300d, 300e, 300f, 300g) selon la revendication 5, dans lequel pour chacune des première et seconde régions de bord (348a, 348b), une première couche des couches pliées (360a, 360b, 360c, 360d, 360e, 360f, 360g, 360h, 360i, 360j) est liée à une deuxième couche des couches pliées (360a, 360b, 360c, 360d, 360e, 360f, 360g, 360h, 360i, 360j) par un adhésif.

8. Article absorbant (300a, 300b, 300c, 300d, 300e, 300f, 300g) selon l'une quelconque des revendications 1 à 7, dans lequel le premier feuillet de substrat comprend un tissu.

9. Article absorbant (300a, 300b, 300c, 300d, 300e, 300f, 300g) selon la revendication 8, dans lequel le deuxième feuillet de substrat comprend un non-tissé et, pour la couche inférieure (356), le deuxième feuillet de substrat est configuré pour être disposé plus près d'un porteur que ne l'est le premier feuillet substrat lorsque l'article absorbant est porté.

10. Article absorbant (300a, 300b, 300c, 300d, 300e, 300f, 300g) selon l'une quelconque des revendications 1 à 9, dans lequel le stratifié (332) est plié de telle sorte que, pour chacune des première et seconde régions de bord (348a, 348b) :
une couche la plus inférieure des couches pliées (360a, 360b, 360c, 360d, 360e, 360f, 360g, 360h, 360i, 360j) a des première et seconde portions et s'étend latéralement entre celles-ci, la première portion étant disposée plus près de l'autre des régions de bord (348a, 348b) que ne l'est la seconde portion ; et
la première portion est configurée pour être disposée sur la couche inférieure (356) lorsque le châssis (304) est dans la configuration portable.

11. Article absorbant (300a, 300b, 300c, 300d, 300e, 300f, 300g) selon l'une quelconque des revendications 1 à 10, dans lequel le SAP de chacun des feuillets absorbants (340) a une capacité de rétention centrifuge entre 33 et 52 grammes par gramme (g/g).

12. Article absorbant (300a, 300b, 300c, 300d, 300e, 300f, 300g) selon l'une quelconque des revendications 1 à 11, dans lequel le SAP de chacun des feuillets absorbants (340) a un poids de base entre 60 et 75 grammes par mètre carré (g/m²).

13. Article absorbant (300a, 300b, 300c, 300d, 300e, 300f, 300g) selon l'une quelconque des revendications 1 à 12, comprenant :
un insert (412) s'étendant longitudinalement, couplé au stratifié (332) de telle sorte que pour chacune des première et seconde régions de bord (348a, 348b), une portion de l'insert (412) est disposée au-dessus de la couche inférieure (356) et en dessous d'au moins une des couches pliées (360a, 360b, 360c, 360d, 360e, 360f, 360g, 360h, 360i, 360j) ;
dans lequel l'insert (412) comprend au moins un parmi :
des peluches et du SAP ; et
un non-tissé polymère lié à l'air.

14. Article absorbant (300a, 300b, 300c, 300d, 300e, 300f, 300g) selon l'une quelconque des revendications 1 à 13, dans lequel :
les deux feuillets de substrat ou plus comprennent trois feuillets de substrat ou plus ;
les un ou plusieurs feuillets absorbants (340) comprennent deux feuillets absorbants (340) ou plus ; et
un deuxième feuillet des feuillets absorbants (340) est disposé entre le deuxième feuillet de substrat et un troisième feuillet des feuillets de substrat.

15. Article absorbant (300a, 300b, 300c, 300d, 300e, 300f, 300g) selon l'une quelconque des revendications 1 à 14, dans lequel chacune des première et seconde régions de bord (348a, 348b) couvre au moins 20 % de la largeur (344) de l'âme absorbante (328).
